# EUROPEAN PATENT APPLICATION

(11) **EP 2 679 267 A1**
(43) Date of publication of application: **01.01.2014**
(21) Application number: 12174256.3
(22) Date of filing: 29.06.2012
(51) Int. Cl.: A61M 16/06

(54) **Nasal cushion with pivotable prongs for a respiratory nasal mask**

(71) Applicant: Air Liquide Medical Systems, 92160 Antony (FR)
(72) Inventor: Alberici, Luca, 25128 Brescia (IT); Masserdotti, Fulvio, 25075 Brescia (IT); Sandoni, Giuseppe, 25124 Brescia (IT)
(74) Representative: Pittis, Olivier

(57) **Abstract**

The invention concerns a nasal cushion for a respiratory nasal mask comprising a hollow body (11) with an internal chamber (2), and a pair of nasal prongs (12) each connected by a proximal end (19), to the hollow body (11) and each comprising an internal gas passage in fluid communication with the internal chamber (2) of the hollow body (11), each of said nasal prongs (12) further having a distal end (9) comprising an outlet orifice (13) fluidly communicating with the internal gas passage. Further, the proximal end (19) of the nasal prongs (12) comprises a flexible bellow portion (10) allowing each prong (12) to pivot so as to modify its orientation with respect to the hollow body (11). The invention also concerns a respiratory nasal mask equipped with such a nasal cushion and its use for treating respiratory failures or disorders, such as sleep apnoea or the like.

## Description

The invention concerns a nasal cushion for a respiratory mask that allows the mask to auto-adapt on the patient's face, and a nasal mask equipped with such a nasal cushion.

Nasal pillow masks are used for non invasive ventilation for treating or preventing acute respiratory failures or disorders, as can occur, for instance, for sleep apnoea disorders or the like.

Such a mask generally comprises a rigid or semi-rigid hollow shell or mask body defining an internal breathing chamber or volume, wherein a respiratory gas, such as air under pressure, is introduced via an inlet port through a short tube assembly to which is connected a gas feeding line by means of a tubular connector.

The rigid or semi-rigid mask body is fixed to a headgear for correctly positioning, maintaining and/or securing the mask on the head of a patient.

The mask body further comprises a peripheral border which is designed to couple with a nasal pillow cushion or nasal cushion.

Nasal cushions are commonly made of soft material, such as silicone, and comprise two prongs or cannulae that are configured to engage with the internal contour of the nostrils when being partially inserted into the nostrils of the patient.

Examples of nasal pillow masks equipped with nasal cushions are given by documents EP-A-2140902, EP-A-1603619, EP-A-2051761, EP-A-2259827, EP-A-2349428 and WO-A-2009/151344.

As nasal pillow masks are used for the treatment or prevention of acute respiratory failures, they are usually worn overnight and even during the day by the patients, and quality of treatment and comfort of use are hence very important features.

However, as the morphology of the face is different from one patient to another, it is often difficult with existing masks to correctly position the nasal cushion, especially the pair of prongs, into the nostrils of the patient and/or to maintain the mask in a suitable desired position.

In other words, a main problem that remains with existing nasal masks is that it is often difficult to achieve an efficient auto-positioning of the nasal mask into the patient's nostrils, especially concerning the pair of prongs, so as to avoid gas leaks and ensure a good gas distribution and consequently an efficient therapy.

Hence, the main goal of the present invention is to provide an improved nasal cushion and mask.

The solution of the present invention is a nasal cushion comprising a hollow body with an internal chamber, and a pair of nasal prongs each connected by a proximal end, to the hollow body and each comprising an internal gas passage in fluid communication with the internal chamber of the hollow body, each of said nasal prongs further having a distal end comprising an outlet orifice fluidly communicating with the internal gas passage, **characterized in that** the proximal end of the nasal prongs comprises a flexible bellow portion allowing each prong to pivot so as to modify its orientation with respect to the hollow body.

Depending on the considered embodiment, the nasal cushion of the present invention can comprise one or more of the following features :
- each flexible bellow portion comprises at least one recess, preferably said at least one recess has an annular shape.
- said at least one recess corresponds to a radial projection of the peripheral wall of said proximal end of the nasal prongs into the internal gas passages of said nasal prongs.
- the flexible bellow portions are located at the proximal end of the nasal prongs.
- each nasal prong comprises, at said distal end, an enlarged head comprising the outlet orifice.
- said enlarged heads have a generally tronconical or quasi-tronconical shape.
- the enlarged heads are made of a soft deformable material, preferably of silicone.
- the hollow body and the pair of nasal prongs are made of silicone.
- each bellow portion comprises at least one recess forming an inclination angle β of between -60 and +60°, the inclination angle β being the angle formed between the plane of coupling between the main body and the proximal end 19 of the nasal prongs (12), i.e. the rest of the nasal cushion, and the plane of direction of the development axis of the bellow portion.
- each bellow portion comprises at least one recess further forming an opening angle α of between 10° and 150 °, which is the opening angle of the bellow, i.e. the angle formed by the opposite surfaces (considered in cross section) of the groove or recess of the proximal end of each prongs and which further forms at least a part of the bellow
- each bellow portion comprises one recess forming a groove extending over the entire circumference of the proximal end of the nasal prongs.
- each bellow portion comprises several recesses, preferably the recesses are arranged such as to be in successive parallel planes.
- the hollow body comprises fixation means for fixing the nasal cushion to a mask body.

The present invention also concerns a nasal respiratory mask comprising a mask body and a nasal cushion according to the present invention, and further comprising a headgear with straps or similar.

The present invention will be better understood thanks to the following description made in reference to the accompanying drawings among which :
- Figure 1 shows an embodiment of a nasal mask according to the present invention,
- Figure 2 represents the body and part of the headgear of the nasal mask of Figure 1,
- Figure 3 represents an embodiment of a nasal cushion according to the present invention that equips the mask of Figure 1,
- Figure 4 is a view in cross section of the nasal cushion of Figure 3, and
- Figure 5 shows several possible embodiments of nasal cushions according to the present invention.

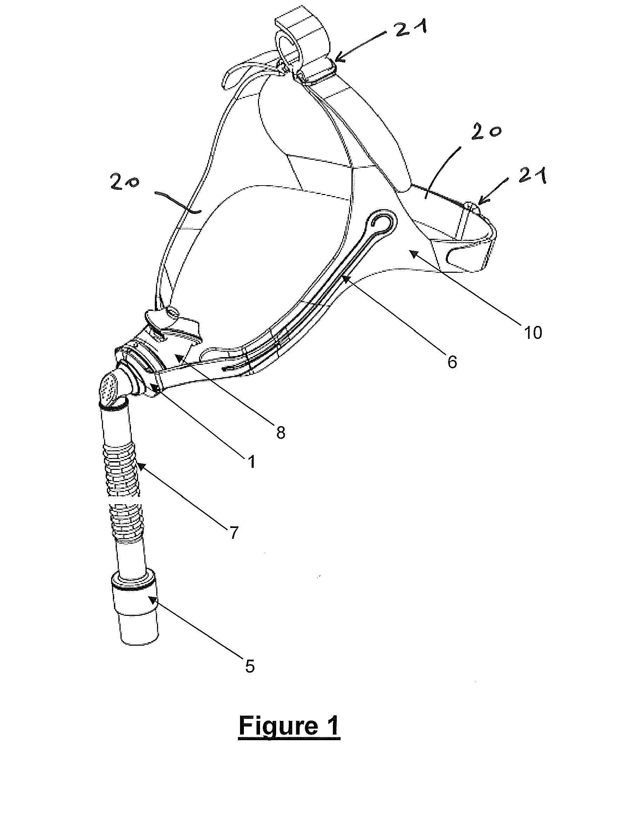

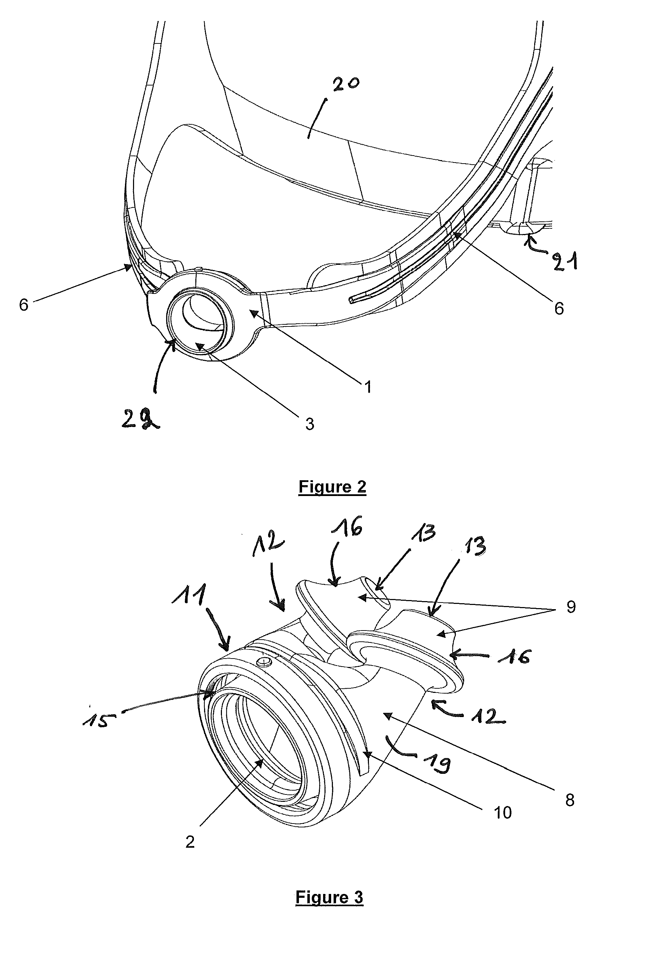

As shown in Figures 1 and 2, the nasal pillow mask according to the present invention comprises a rigid or semi-rigid hollow shell or mask body 1 defining an internal breathing chamber 2 or volume, wherein a respiratory gas, such as air under pressure, is introduced via an inlet port 3 by means of a flexible tube assembly 7 that is connected to a gas feeding line (non shown) thanks to a tubular connector 5.

The gas inlet orifice 3 is arranged at the centre of the mask body 1 and through its wall thereby allowing air under pressure to be introduced in the breathing chamber 2.

The rigid or semi-rigid mask body 1 further comprises two lateral arms 6 which extend away form the mask body 1, and are fixed to a headgear 10 comprising flexible straps 20, typically made of fabric, and connecting elements 21, such as buckles or similar.

The mask body 1 is preferably made of a polymer material, such as polycarbonate (PC), polypropylene (PP), ABS, nylon or polystyrene (PS).

The mask body 1 further comprises a peripheral border 22 which is designed to couple with fixation means 15 arranged on the nasal cushion 8 so that the nasal 8 cushion can be made integral with the mask body 1 and no gas leaks appear in between.

The fixation means 15 comprise the peripheral border of the hollow body 11 of the nasal cushion 8 that is configured so as to match the peripheral border of the mask body 1, i.e. the peripheral border of the hollow body 11 of the cushion and the corresponding one of the mask body 1 have profiles or shapes that cooperate together so as to provide a efficient connection between mask body 1 and the hollow body 11 of the cushion.

Details of the nasal cushion 8 according to the present invention are shown in Figure 3. It comprises a hollow body 11 with an internal chamber 2, and a pair of nasal prongs 12 connected by their proximal end 19, to the hollow body 11. For example, the nasal prongs 12 and the hollow body 11 can be moulded in one piece.

Each nasal prong 12 is a kind of nasal cannula or small tube comprising an internal gas passage in fluid communication with the internal chamber 2 of the hollow body 11 so as to be able to convey and distribute gas under pressure to the patient's nostrils. The gas is delivered by each prong 12 by an outlet orifice 13 arranged at its distal end 9, which is in fluid communication with the internal gas passage of the prong 12.

According to the present invention, for ensuring an efficient and auto-positioning of the nasal mask into the patient's nostrils, especially a correct positioning and orientation of the pair of prongs 12 that avoids gas leaks and ensures a good gas distribution and consequently an efficient therapy, the proximal end 19 of the nasal prongs 12 comprises a flexible bellow portion 10 allowing each prong 12 to pivot with respect to the hollow body 11.

The flexible bellow portions 10 are located at the proximal end 19 of the nasal prongs 12, i.e. in the vicinity of the hollow body 11.

Thanks to the flexible bellow portion 10, the nasal prongs 12 are pivotable and flexible with respect to the hollow body 11 of the cushion and to the mask body 1. For allowing the nasal prongs 8 to efficiently pivot, i.e. to be mobile so that can bend, bow, oscillate or similar, while being maintained by their proximal end 19, with respect to the cushion hollow body 11, the nasal prongs 8, including their flexible bellow portion 10, are preferably made of a flexible material, such as thermoplastic rubber or elastomeric polymer. Preferably, they are made of silicone or the like.

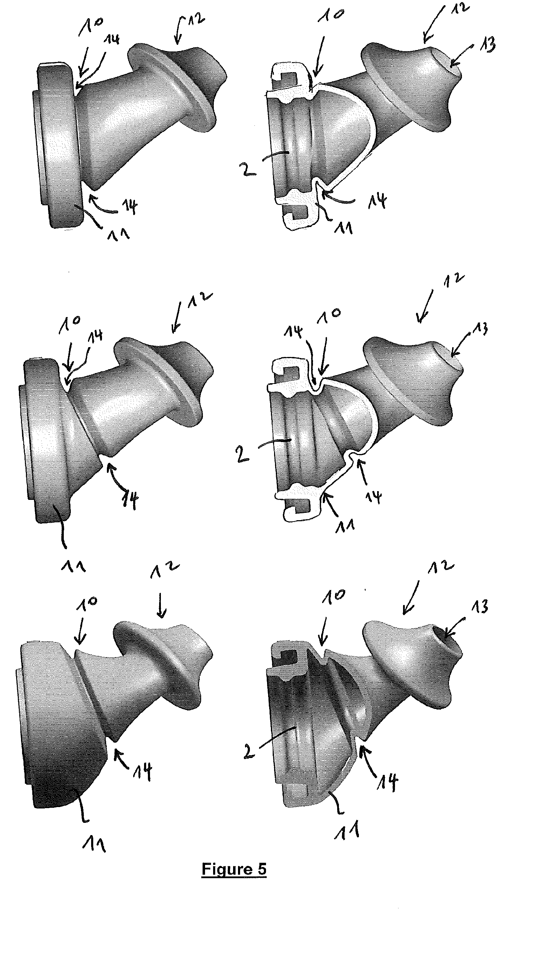

The flexible bellow portion 10 of each prong 12 comprises one (or several) recess 14 having preferably an annular or semi-annular shape. In other words, the recess 14 forms a groove in the wall of the proximal end 19, preferably extending over the entire external circumference of the proximal end 19 as shown in Figure 5.

Actually, each recess 14 corresponds to a radial projection of the peripheral wall of said proximal end 19 into the internal gas passages of said proximal end 19. The bellow portion 10 comprising the groove(s) or recess(es) 14 is preferably made by moulding.

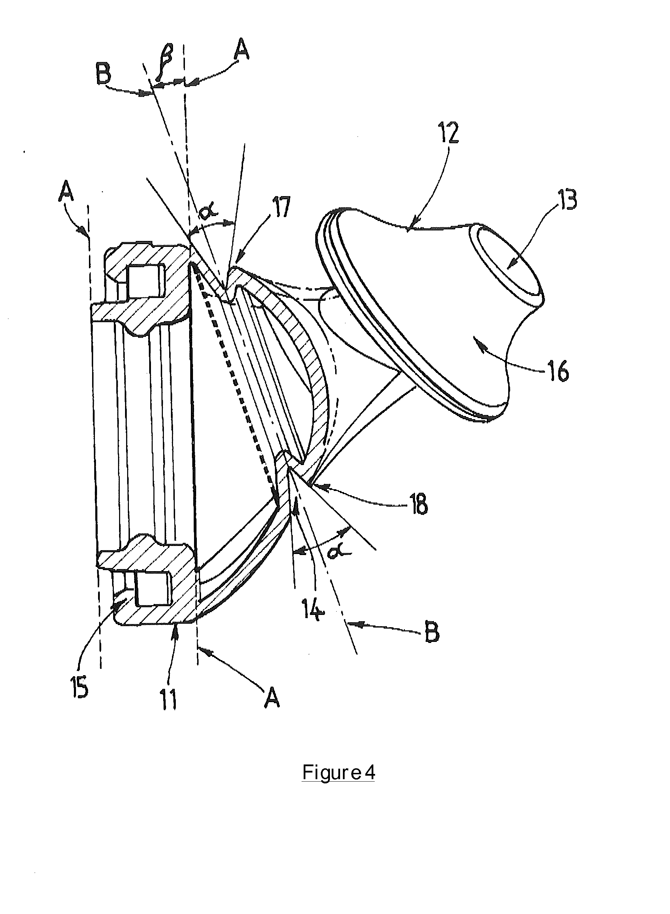

As detailed in Figure 4, the recess 14 or groove of each bellow portion 10, forms an inclination angle β of between -60° and +60°, whereas the recess 14 or groove of said bellow portion 10, forms an opening angle α of between 10 and 150 °, wherein :
- the inclination angle β is the angle formed between planes A and B, A being the plane of coupling between the main body 1 and the rest of the cushion 8, especially the proximal end 19 of each of the nasal prongs 12, and B being the plane of direction of the development axis of the bellow portion, and
- the opening angle α is the opening angle of the bellow, i.e. the angle formed by the opposite surfaces (considered in cross section as shown on Fig. 4) of the groove or recess 14 of the proximal end 19 of each prongs and forming at least a part of the bellow.

Actually, the inclination angle β and the opening angle α can be equal or different.

Preferably, the recess 14 in the top area 17 and the one in the bottom area 18 form a continuous groove extending over the entire external periphery of the proximal end 19.

Further, each nasal prong 12 of the nasal pillow cushion 8 comprises, at its distal end 9, an enlarged head 16 configured to have a generally tronconical or quasi-tronconical shape as shown in Figures 3-5.

Preferably, said enlarged heads 16 have an elliptical surface 9 and are made of a thin resilient material, such as a thin silicone wall, so that the enlarged heads 16 inflate like a balloon and are deformed by the gas pressure exerted on their internal wall, when a pressurized gas passes through them, thereby creating an efficient seal between the surface 9 of each enlarged head 16 and the inner wall of the nostrils of the patient. Indeed, when the enlarged heads 16 are inflated by gas, they match the inner contour of the patient's nostrils, thus creating a seal.

The nasal mask and cushion according to the present invention are useable for treating respiratory failures or disorders, such as sleep apnoea or the like.

## Claims

1. Nasal cushion comprising a hollow body (11) with an internal chamber (2), and a pair of nasal prongs (12) each connected by a proximal end (19), to the hollow body (11) and each comprising an internal gas passage in fluid communication with the internal chamber (2) of the hollow body (11), each of said nasal prongs (12) further having a distal end (9) comprising an outlet orifice (13) fluidly communicating with the internal gas passage, **characterized in that** the proximal end (19) of the nasal prongs (12) comprises a flexible bellow portion (10) allowing each prong (12) to pivot so as to modify its orientation with respect to the hollow body (11).

2. Nasal cushion according to the preceding Claim, **characterized in that** each flexible bellow portion (10) comprises at least one recess (14), preferably said at least one recess (14) has an annular shape.

3. Nasal cushion according to any one of the preceding Claims, **characterized in that** said at least one recess (14) corresponds to a radial projection of the peripheral wall of said proximal end (19) into the internal gas passages of said nasal prongs (12).

4. Nasal cushion according to any one of the preceding Claims, **characterized in that** the flexible bellow portions (10) are located at the proximal end (19) of the nasal prongs (12).

5. Nasal cushion according to any one of the preceding Claims, **characterized in that** each bellow portion (10) comprises at least one recess (14) forming an inclination angle (β) of between -60 and +60°, the inclination angle (β) being the angle formed between the plane (A) of coupling between the main body (11) and the proximal end (19) of the nasal prongs (12), and the plane of direction of the development axis of the bellow portion (10).

6. Nasal cushion according to any one of the preceding Claims, **characterized in that** each bellow portion (10) comprises at least one recess (14) forming an opening angle (α) of between 10° and 150 °, the opening angle (α) being the opening angle of the the recess (8) of the bellow portion (10) at the proximal end of each prongs (12).

7. Nasal cushion according to any one of the preceding Claims, **characterized in that** each nasal prong (12) comprises, at said distal end (9), an enlarged head (16) comprising the outlet orifice (13).

8. Nasal cushion according to any one of the preceding Claims, **characterized in that** said enlarged heads (16) have a generally tronconical or quasi-tronconical shape.

9. Nasal cushion according to any one of the preceding Claims, **characterized in that** the enlarged heads (16) are made of a soft deformable material, preferably of silicone.

10. Nasal cushion according to any one of the preceding Claims, **characterized in that** the hollow body (11) and the pair of nasal prongs (12) are made of silicone.

11. Nasal cushion according to any one of the preceding Claims, **characterized in that** each bellow portion (10) comprises one recess (14) forming a groove extending over the entire circumference of the proximal end (19).

12. Nasal cushion according to any one of the preceding Claims, **characterized in that** the hollow body (11) comprises fixation means (15) for fixing the nasal cushion to a mask body (1).

13. Nasal respiratory mask comprising a mask body (1) and a nasal cushion according to any one of the preceding Claims.

14. Nasal respiratory mask according to Claim 13, **characterized in that** it further comprises a headgear (10).

## Amended claims

### Amended claims in accordance with Rule 137(2) EPC.

**1.** Nasal cushion comprising a hollow body (11) with an internal chamber (2), and a pair of nasal prongs (12) each connected by a proximal end (19), to the hollow body (11) and each comprising an internal gas passage in fluid communication with the internal chamber (2) of the hollow body (11), each of said nasal prongs (12) further having a distal end (9) comprising an outlet orifice (13) fluidly communicating with the internal gas passage, **characterized in that** :
- the proximal end (19) of the nasal prongs (12) comprises a flexible bellow portion (10) allowing each prong (12) to pivot so as to modify its orientation with respect to the hollow body (11),
- the nasal prongs (12) and the hollow body (11) are moulded in one piece,
- fixation means (15) for fixing the nasal cushion to a mask body (1) and comprising the peripheral border of the hollow body (11) of the nasal cushion (8), are arranged on the nasal cushion (8),
- the hollow body (11) and the pair of nasal prongs (12) are made of silicone, and
- the peripheral border of the hollow body (11) has a circular shape.

**2.** Nasal cushion according to the preceding Claim, **characterized in that** each flexible bellow portion (10) comprises at least one recess (14), preferably said at least one recess (14) has an annular shape.

**3.** Nasal cushion according to any one of the preceding Claims, **characterized in that** said at least one recess (14) corresponds to a radial projection of the peripheral wall of said proximal end (19) into the internal gas passages of said nasal prongs (12).

**4.** Nasal cushion according to any one of the preceding Claims, **characterized in that** the flexible bellow portions (10) are located at the proximal end (19) of the nasal prongs (12).

**5.** Nasal cushion according to any one of the preceding Claims, **characterized in that** each nasal prong (12) comprises, at said distal end (9), an enlarged head (16) comprising the outlet orifice (13).

**6.** Nasal cushion according to any one of the preceding Claims, **characterized in that** said enlarged heads (16) have a generally tronconical or quasi-tronconical shape.

**7.** Nasal cushion according to any one of the preceding Claims, **characterized in that** the enlarged heads (16) are made of a soft deformable material, preferably of silicone.

**8.** Nasal cushion according to any one of the preceding Claims, **characterized in that** each bellow portion (10) comprises one recess (14) forming a groove extending over the entire circumference of the proximal end (19).

**9.** Nasal respiratory mask comprising a mask body (1) and a nasal cushion according to any one of the preceding Claims.

**10.** Nasal respiratory mask according to Claim 9, **characterized in that** it further comprises a headgear (10).

**11.** Nasal respiratory mask according to Claim 9, **characterized in that** it further comprises a peripheral border (22) designed to couple with the fixation means (15) arranged on the nasal cushion (8) so that the nasal (8) cushion can be made integral with the mask body (1) and no gas leaks appear in between.

**12.** Nasal respiratory mask according to Claim 9 or 11, **characterized in that** the peripheral border of the hollow body (11) of the nasal cushion (8) is configured so as to match the peripheral border of the mask body (1).
